(19) Europäisches Patentamt / European Patent Office / Office européen des brevets

(11) EP 3 295 969 A1

(12) EUROPEAN PATENT APPLICATION

(43) Date of publication:
21.03.2018 Bulletin 2018/12

(51) Int Cl.:
A61L 31/02 (2006.01)  A61L 31/08 (2006.01)
A61L 29/02 (2006.01)  A61L 29/10 (2006.01)
A61L 29/12 (2006.01)  A61L 31/12 (2006.01)
A61L 29/18 (2006.01)  A61L 31/18 (2006.01)

(21) Application number: 17275145.5

(22) Date of filing: 19.09.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 20.09.2016 US 201662397123 P

(71) Applicant: Cook Medical Technologies LLC
Bloomington, IN 47404 (US)

(72) Inventors:
• BUTLER, James
  Aherlow, Co. Tipperary (IE)
• NEILAN, John
  Gort, Co. Galway (IE)

(74) Representative: Williams Powell
11 Staple Inn
London WC1V 7QH (GB)

(54) RADIOPAQUE COMPOSITE WIRE FOR MEDICAL APPLICATIONS AND METHOD OF MAKING A RADIOPAQUE COMPOSITE WIRE

(57) A radiopaque composite wire for medical applications comprises a core (102) comprising a rare earth metal, an outer layer (104) comprising a nickel-titanium alloy disposed over the core (102), and a diffusion barrier (106) comprising a barrier material between the core (102) and the outer layer (104). A method of making a radiopaque composite wire includes cold drawing a composite billet (200) through a die, where the composite billet includes a tube (204) comprising a nickel-titanium alloy disposed about a rod comprising a rare earth metal, and a barrier layer (206) comprising a barrier material disposed between the tube and the rod. After cold drawing, the composite billet (200) is annealed to relieve strain. After multiple passes of the cold drawing and annealing, a radiopaque composite wire having a core comprising the rare earth metal, an outer layer comprising the nickel-titanium alloy, and a diffusion barrier comprising the barrier material between the core and the outer layer is formed.

FIG. 1

## Description

TECHNICAL FIELD

The present disclosure is related generally to biocompatible wire for medical applications and more particularly to a radiopaque composite wire.

BACKGROUND

Nickel-titanium alloys are commonly used for the manufacture of intraluminal biomedical devices, such as self-expandable stents, stent grafts, embolic protection filters, and stone extraction baskets. Such devices may exploit the superelastic or shape memory behavior of equiatomic or near-equiatomic nickel-titanium alloys, which are commonly referred to as Nitinol.

As a result of the poor radiopacity of nickel-titanium alloys, however, such devices may be difficult to visualize from outside the body using non-invasive imaging techniques, such as x-ray fluoroscopy. Visualization is particularly problematic when the intraluminal device is made of fine wires or thin-walled struts. Consequently, a clinician may not be able to accurately place and/or manipulate a Nitinol stent or basket within a body vessel.

Traditional approaches to improving the radiopacity of nickel-titanium medical devices include the use of radiopaque markers or coatings. For example, gold markers attached to ends of a stent may guide the positioning of the device and delineate its length during an x-ray procedure. Alternatively, a medical device may be plated, clad or otherwise coated with gold or another heavy metal to create a radiopaque surface or outer layer. In another approach, a dense cylinder comprising a metal such as gold or platinum may be included within the lumen of a stent to produce a radiopaque core. These approaches to improving radiopacity may have shortcomings, however. In some cases, markers may be easily dislodged or may undesirably increase the delivery profile of the device, and radiopaque cores comprising noble metals may be expensive to fabricate.

BRIEF SUMMARY

Aspects of the invention seek to provide an improved radiopaque composite wire, radiopaque medical device, and/or method.

According to an aspect of the invention, there is provided a radiopaque composite wire as in claim 1.

According to an aspect of the invention, there is provided a radiopaque composite wire for medical applications, the radiopaque composite wire comprising:

a core comprising a rare earth metal;
an outer layer comprising a nickel-titanium alloy disposed over the core; and
a diffusion barrier comprising a barrier material between the core and the outer layer.

In some embodiments, the barrier material is selected from the group consisting of: a refractory metal, a rare earth oxide, an iron-based material, and carbon.

In some embodiments, the barrier material comprises a refractory metal selected from the group consisting of: Nb, Mo, Ta, W, Re, Ti, V, Cr, Zr, Hf, Ru, Rh, Os and/or Ir.

In some embodiments, the nickel-titanium alloy is superelastic with an austenite finish temperature ($A_f$) at or below body temperature.

In some embodiments, the rare earth metal is selected from the group consisting of: Sc, Y, Ce, La, Pr, Nd, Pm, Sm, Eu, Gd, Tb, Dy, Ho, Er, Tm, Yb, Lu, Ac, Th, Pa and U.

In some embodiments, the rare earth metal is selected from the group consisting of: Sc, Y, La, Lu and Yb, the composite wire exhibiting MRI compatibility.

In some embodiments, the core further comprises an additional element selected from the group consisting of: Ag, Cu, Au, Ir and Rh.

In some embodiments, the diffusion barrier comprises a thickness of from about 1 micron to about 50 microns.

In some embodiments, a volume percentage of the core comprising the rare earth metal lies in the range from about 5 vol.% to about 60 vol.%.

According to an aspect of the invention, there is provided a radiopaque medical device comprising:

at least one radiopaque composite wire comprising:

a core comprising a rare earth metal;
an outer layer comprising a nickel-titanium alloy disposed over the core; and
a diffusion barrier comprising a barrier material between the core and the outer layer,

the radiopaque medical device being an insertable and/or implantable medical device for use in a body vessel.

In some embodiments, the radiopaque medical device is selected from the group consisting of: a wire guide, a stent, a stent graft, a torqueable catheter, an introducer sheath, a radiopaque marker or marker band, a grasper, a snare, a basket, a vascular plug, and an embolic protection filter.

In some embodiments, the radiopaque medical device is a stent selected from the group consisting of: biliary stent, enteral stent, duodenal stent, colonic stent, and esophageal stent.

According to an aspect of the invention, there is provided a method as in claim 9.

According to an aspect of the invention, there is provided a method of making a radiopaque composite wire for medical applications, the method comprising:

cold drawing a composite billet through a die, the composite billet comprising: a tube comprising a nickel-titanium alloy disposed about a rod comprising a rare earth metal, and a barrier layer comprising a barrier material disposed between the tube and the rod;

after cold drawing, annealing the composite billet to relieve strain, and

after multiple passes of the cold drawing and annealing, forming a radiopaque composite wire having a core comprising the rare earth metal, an outer layer comprising the nickel-titanium alloy, and a diffusion barrier comprising the barrier material between the core and the outer layer.

In some embodiments, during cold drawing, at least about 7.5% cold work is imparted to the composite billet.

In some embodiments, the method further comprises, prior to cold drawing the composite billet, hot working the composite billet.

In some embodiments, the method further comprises, prior to hot working the composite billet, fabricating the composite billet, wherein fabricating the composite billet comprises:

drilling a longitudinal hole through an ingot comprising the nickel-titanium alloy to form the tube; and assembling the barrier layer comprising the barrier material and the rod comprising the rare earth metal in the tube.

In some embodiments, assembling the barrier layer and the rod in the tube comprises:

wrapping the barrier layer about the rod, the barrier layer comprising a foil, and inserting the barrier layer and rod into the tube.

In some embodiments, assembling the barrier layer and the rod in the tube comprises:

applying the barrier layer onto the rod using a vapor or electrochemical deposition process, and inserting the rod coated with the barrier layer into the tube.

In some embodiments, assembling the barrier layer and the rod in the tube comprises:

inserting the rod into the tube; and inserting a hollow cylinder comprising the barrier material into the tube.

In some embodiments, assembling the barrier layer and the rod in the tube comprises:

applying the barrier layer onto an inner wall of the tube using a vapor or electrochemical deposition process, and inserting the rod into the tube.

In some embodiments, the barrier material is selected from the group consisting of: a refractory metal, a rare earth oxide, an iron-based material, and carbon.

In embodiments, a radiopaque composite wire for medical applications has a core comprising a rare earth metal, an outer layer comprising a nickel-titanium alloy disposed over the core, and a diffusion barrier comprising a barrier material between the core and the outer layer.

In some embodiments, a radiopaque medical device comprises at least one radiopaque composite wire including a core comprising a rare earth metal, an outer layer comprising a nickel-titanium alloy disposed over the core, and a diffusion barrier comprising a barrier material between the core and the outer layer. The radiopaque medical device can be an insertable and/or implantable medical device for use in a body vessel.

In some embodiments, a method of making a radiopaque composite wire includes cold drawing a composite billet through a die, where the composite billet comprises: a tube comprising a nickel-titanium alloy disposed about a rod comprising a rare earth metal, and a barrier layer comprising a barrier material disposed between the tube and the rod. After cold drawing, the composite billet can be annealed to relieve strain. After multiple passes of the cold drawing and annealing, a radiopaque composite wire having a core comprising the rare earth metal, an outer layer comprising the nickel-titanium alloy, and a diffusion barrier comprising the barrier material between the core and the outer layer can be formed.

BRIEF DESCRIPTION OF THE DRAWINGS

Embodiments are described below, by way of example only, with reference to the accompanying drawings.

FIG. 1 provides a schematic of an exemplary radiopaque composite wire.

FIG. 2 is a schematic of an exemplary cold drawing process to form the composite wire from a composite billet.

FIG. 3 is a scanning electron microscope (SEM) image of a transverse cross-section of a hot rolled composite billet formed from a nickel-titanium alloy tube over an erbium rod with a niobium foil in between.

FIG. 4 is a SEM image showing a transverse cross-section of a radiopaque composite wire comprising a nickel-titanium alloy outer layer, an erbium core, and a niobium diffusion barrier in between.

FIG. 5 is a SEM image of a transverse cross-section of a hot rolled composite billet formed from a nickel-titanium alloy tube over a lutetium rod with a niobium foil in between.

FIG. 6 is a SEM image showing a transverse cross-section of a radiopaque composite wire comprising a nickel-titanium alloy outer layer, a lutetium core, and a niobium diffusion barrier in between.

FIG. 7 is a stress-strain curve obtained from a uniaxial tensile test of the radiopaque composite wire shown in FIG. 4.

FIGs. 8A-8F show x-ray images with phantom of the radiopaque composite wires of FIGs. 4 and 6 in comparison with binary NiTi wires and a Pt core (30 vol.%) wire.

FIG. 9 is a schematic of an exemplary stent woven from the radiopaque composite wires described herein.

DETAILED DESCRIPTION

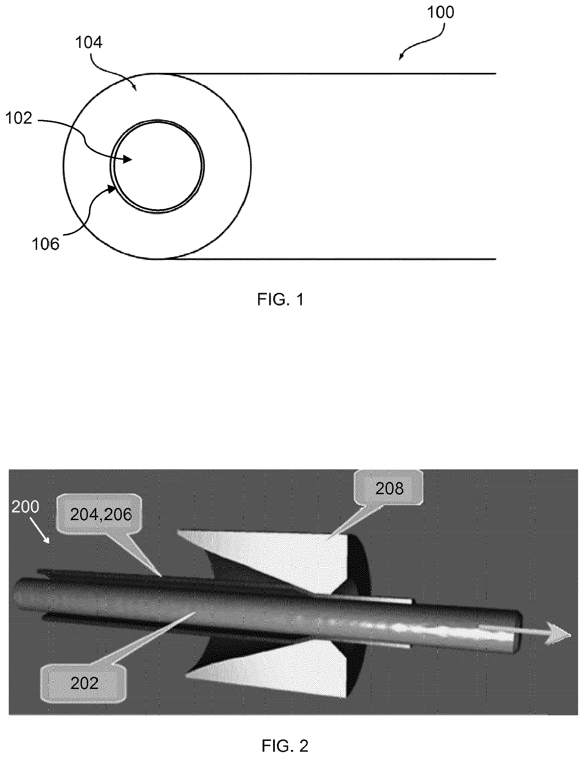

Referring to FIG. 1, a radiopaque composite wire 100 for medical applications includes a core 102 comprising a rare earth metal, an outer layer 104 comprising a nickel-titanium alloy disposed over the core 102, and a diffusion barrier 106 between the core 102 and the outer layer 104. The diffusion barrier 106 comprises a barrier material that inhibits or prevents diffusion of nickel from the outer layer 104 into the core 102. Diffusion of nickel into the core can embrittle the rare earth metal and may also degrade the properties of the nickel-titanium alloy. Barrier materials that may be effective for inhibiting nickel diffusion include refractory metals, rare earth oxides, iron-based materials and/or carbon (e.g., graphite). The presence of the diffusion barrier 106 may be particularly important during processing of the composite wire 100.

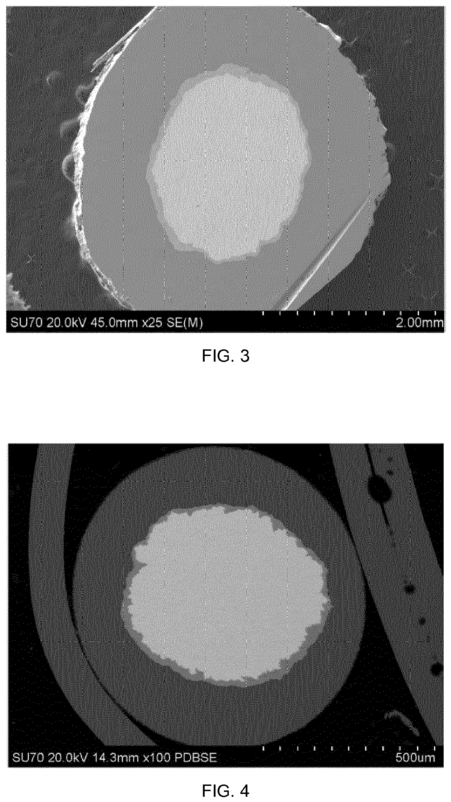

Advantageously, the rare earth metal of the core 102 is highly radiopaque while also being compatible with magnetic resonance imaging (MRI). A radiopaque material preferentially absorbs incident x-rays and tends to show high radiation contrast and good visibility in x-ray images. MRI compatible materials can be viewed *in vivo* in MRI scans and images without significant artifacts. The radiopacity of several rare earth elements has been shown to be comparable to that of platinum - a highly radiopaque metal - over the photon energy range from about 40 keV to about 80 keV, as shown by the absorption coefficient data presented in FIGs. 2 and 3. Lutetium, which has a higher density than the rare earth elements examined in these figures, is expected to have an even higher radiopacity.

The nickel-titanium alloy of the outer layer 104 may exhibit superelastic and/or shape memory behavior. That is, the nickel-titanium alloy may undergo a phase transformation that allows it to "remember" and return to a previous shape or configuration. More specifically, the nickel-titanium alloy may transform between a lower temperature phase (martensite) and a higher temperature phase (austenite). Austenite is characteristically the stronger phase, and martensite may be deformed up to a recoverable strain of about 8%. Strain introduced in the alloy in the martensitic phase to achieve a shape change may be substantially recovered upon completion of a reverse phase transformation to austenite, allowing the alloy to return to a previous shape. The strain recovery may be driven by the application and removal of stress (superelastic effect) and/or by a change in temperature (shape memory effect). Such alloys are commonly referred to as Nitinol or Nitinol alloys, and they are typically near-equiatomic in composition. Due to the presence of the Ni-Ti alloy outer layer 104, the radiopaque composite wire 100 may behave superelastically and/or utilize the shape memory effect.

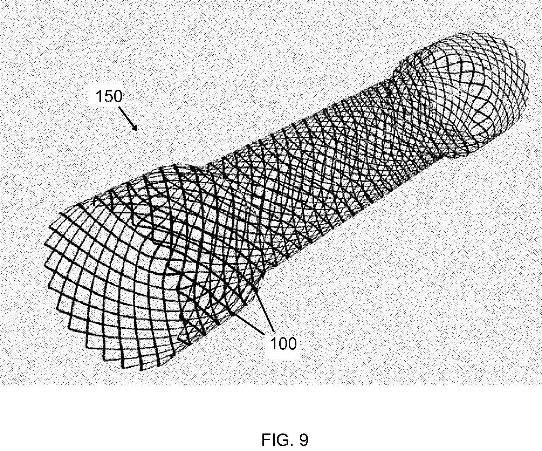

Radiopaque composite wires as described herein may find use in any of a number of medical devices. For example, the radiopaque composite wire may be employed individually or in combination as part of an insertable or implantable medical device, such as, for example, a wire guide, a stent, a stent graft, a torqueable catheter, an introducer sheath, an orthodontic arch wire, a radiopaque marker or marker band, or a manipulation, retrieval, or occlusive device such as a grasper, a snare, a basket (e.g., stone extraction or manipulation basket), a vascular plug, or an embolic protection filter. For example, the radiopaque composite wires may be suitable for stents (e.g., wire-woven stents) that currently utilize wires marked or filled with expensive noble metals for radiopacity. Exemplary stents that may comprise one or more radiopaque composite wires as described herein include biliary stents, enteral stents, duodenal stents, colonic stents, and esophageal stents. A schematic of an exemplary woven stent 150 formed from a plurality of the radiopaque composite wires 100 is provided in FIG. 9.

The rare earth metal of the core 102 may be selected from among the following elements, which are found in the lanthanide and actinide series of the periodic table: La, Ce, Pr, Nd, Pm, Sm, Eu, Gd, Tb, Dy, Ho, Er, Tm, Yb, Lu, Ac, Th, Pa and/or U. Sc and Y, which are sometimes considered rare earth elements, may also be employed as the rare earth metal. Preferred rare earth metals for the core 102 include Er and Lu. The core 102 may comprise more than one rare earth metal and/or other element(s) that can form an alloy or compound with the rare earth metal. Thus, the core 102 may comprise a metal, alloy or compound that includes at least one rare earth element selected from Sc, Y, the lanthanide series elements, and the actinide series elements.

In one example, the core 102 may include, in addition to the rare earth metal, an element selected from among Ag, Cu, Au, Ir and Rh that forms an alloy or compound with the rare earth metal. Such rare earth alloys or compounds may exhibit better mechanical properties than the rare earth metal alone (e.g., increased strength and hardness without a detrimental loss of ductility). A class of rare earth-based intermetallic compounds that may have a good balance of strength and ductility include, for example, yttrium-silver (YAg), yttrium-copper (YCu), dysprosium-copper (DyCu), cerium-silver (CeAg), erbium-silver (ErAg), erbium-gold (ErAu), erbium-copper (ErCu), erbium-iridium (ErIr), holmium-cop-

per (HoCu), neodymium-silver (NdAg), yttrium-iridium (YIr) and yttrium-rhodium (YRh). In other embodiments, the core 102 may include only the rare earth metal and any incidental impurities.

Effective barrier materials for the diffusion barrier 106 may be selected from the refractory metals, rare earth oxides, iron-based materials and carbon. For example, suitable refractory metals may include Nb, Mo, Ta, W, Re, Ti, V, Cr, Zr, Hf, Ru, Rh, Os and/or Ir. Preferred refractory metals may include Nb, Mo, Ta, W, and Re. Rare earth oxides that can be used as barrier materials include lanthanum oxide, cerium oxide, praseodymium oxide, neodymium oxide, samarium oxide, europium oxide, gadolinium oxide, terbium oxide, dysprosium oxide, holmium oxide, erbium oxide, lutetium oxide and/or thulium oxide. Iron-based materials such as pure iron, iron alloys, or iron compounds, as well as carbon in the form of graphite, may also be suitable as barrier materials.

Nickel-rich compositions of the nickel-titanium alloy may be advantageous to ensure that the composite wire 100 exhibits superelastic behavior at body temperature. For use in medical applications in the human body, it is beneficial for the nickel-titanium alloy of the composite wire to have an austenite start temperature $A_s$ below body temperature (e.g., 37°C) and an austenite finish temperature $A_f$ at or below body temperature. As known to those of skill in the art, austenite start temperature ($A_s$) is the temperature at which a phase transformation to austenite begins upon heating for a nickel-titanium alloy exhibiting an austenitic phase transformation, and austenite finish temperature ($A_f$) is the temperature at which the phase transformation to austenite concludes upon heating. Martensite start temperature ($M_s$) is the temperature at which a phase transformation to martensite begins upon cooling for a nickel-titanium alloy exhibiting a martensitic phase transformation, and martensite finish temperature ($M_f$) is the temperature at which the phase transformation to martensite concludes upon cooling.

For example, the nickel-titanium alloy may have from greater than 50 at.% Ni to about 52 at.% Ni, or from about 50.6 at.% Ni to about 50.8 at. % Ni. Titanium and any incidental impurities may account for the balance of the nickel-titanium alloy. In some cases, the nickel-titanium alloy may also include a small amount of an additional alloying element (AAE) (e.g., from about 0.1 at.% AAE to about 10 at.% AAE) to enhance the superelastic or other properties of the nickel-titanium alloy. The additional alloying element may be selected from among B, Al, Cr, Mn, Fe, Co, Cu, Zn, Ga, Ge, Zr, Nb, Mo, Tc, Ru, Rh, Pd, Ag, Cd, In, Sn, Sb, Hf, Ta, W, Re, Os, Ir, Pt, Au, Hg, Tl, Pb, Bi, Po, V, and Mischmetal.

A suitable volume ratio of the rare earth core 102 to the Ni-Ti outer layer 104 may be determined based on the desired radiopacity of the composite wire 100. Commercially available drawn filled tubing (DFT) wire including a nickel-titanium shell and a platinum core may be used as a benchmark, since platinum is highly radiopaque and such wires are employed for medical device applications. In some commercial products, the platinum core may account for about 10 vol.% or about 30 vol.% of the DFT wire. As discussed above, the radiopacity of several rare earth elements has been shown to be comparable to or higher than that of platinum over the photon energy range from about 40 keV to about 80 keV. Assuming a similar or better radiopacity, it is possible to determine a suitable amount of rare earth material to incorporate into the core 102.

For example, a commercial wire including a platinum core (30% by volume) and a Ni-rich (51 at.%Ni-49 at.%Ti) nickel-titanium shell includes about 28.70 at.% Pt, along with 36.65 at.% Ni and 34.65 at.% Ti. Assuming that a comparable atomic percentage of Er or Lu provides the same or better radiopacity than Pt, the desired volume percentage of rare earth metal in a radiopaque composite wire may be calculated. An assumption is made that the thickness of the barrier layer is much smaller than the diameter of the core and the thickness of the outer layer, and thus is not included in these calculations. The core is understood to have a cylindrical shape with the outer layer radially surrounding the core along the length thereof. The results are summarized in the tables below.

In the case of a radiopaque composite wire 100 with a core 102 comprising Er or Lu and an outer layer 104 comprising a Ni-Ti alloy (51 at.% Ni), a preferred volume percentage of the rare earth metal may range from about 18 vol.% to about 46 vol.%, although percentages outside this range may also be used. Generally speaking, to achieve a suitable radiopacity from the radiopaque composite wire described herein, the rare earth core 102 may account for at least about 5 vol.% and up to about 60 vol.% of the composite wire 100, and more typically from about 15 vol.% to about 60 vol.% of the composite wire 100. To exceed the radiopacity of a Ni-Ti wire including a 30 vol.% Pt core, the composite wire 100 may have a rare earth core 102 accounting for at least about 46 vol.% of the composite wire, such as from about 46 vol.% to about 60 vol.%.

As indicated above, the diffusion barrier has a small thickness compared to the diameter of the core and the thickness of the outer layer. For example, the diffusion barrier may have a thickness from about 1 micron to about 50 microns for a composite wire of about 1 mm in diameter.

**Table 1. Biomedical grade binary Nitinol**

| Atom Type | at.% | wt.% | vol.% |
|-----------|------|------|-------|
| Ni | 51.00 | 56.05 | 39.21 |
| Ti | 49.00 | 43.95 | 60.79 |

**Table 2. 30% Pt core DFT compared to Er and Lu of similar radiopacity**

| Atom Type | wt.% | at.% | vol.% |
|---|---|---|---|
| Ni | 22.86 | **36.65** | 27.71 |
| Ti | 17.64 | **34.65** | 42.29 |
| Pt | 59.50 | **28.70** | **30.00** |
| | | | |
| Atom Type | wt.% | at.% | vol.% |
| Ni | 24.99 | 36.65 | 21.19 |
| Ti | 19.28 | 34.65 | 32.33 |
| Er | 55.74 | 28.70 | 46.48 |
| | | | |
| Atom Type | wt.% | at.% | vol.% |
| Ni | 24.36 | 36.65 | 21.57 |
| Ti | 18.79 | 34.65 | 32.91 |
| Lu | 56.85 | 28.70 | 45.53 |

**Table 3. 10% Pt core DFT compared to Er and Lu of similar radiopacity**

| Atom Type | wt.% | at.% | vol.% |
|---|---|---|---|
| Ni | 40.64 | **46.27** | 35.37 |
| Ti | 31.73 | **44.27** | 54.62 |
| Pt | 27.63 | **9.46** | **10.00** |
| | | | |
| Atom Type | wt.% | at.% | vol.% |
| Ni | 42.31 | **46.27** | **32.08** |
| Ti | 33.04 | **44.27** | **49.54** |
| Er | 24.65 | **9.46** | **18.38** |
| | | | |
| Atom Type | wt.% | at.% | vol.% |
| Ni | 41.84 | 46.27 | 32.31 |
| Ti | 32.66 | 44.27 | 49.88 |
| Lu | 25.50 | 9.46 | 17.81 |

To fabricate the radiopaque composite wire described above, a composite billet 200 that includes a tube 204 comprising a nickel-titanium alloy disposed about a rod 202 comprising a rare earth metal, where a barrier layer 206 comprising a barrier material lies between the tube 204 and the rod 202, may be cold-drawn through a die 208, as shown schematically in FIG. 2. The barrier material may comprise a refractory metal, a rare earth oxide, an iron-based material and/or carbon, as described above. During cold drawing, which is carried out at ambient temperature without heating, the composite billet 200 is deformed as it passes through the die 208 from a larger diameter to a smaller diameter (and a longer length).

Cold drawing also typically produces an increase in the strength of the drawn specimen, and a corresponding decrease in ductility, due to the effects of strain hardening. Therefore, the composite billet 200 may benefit from an anneal after cold drawing to relieve strain. Annealing may be carried out at a temperature in the range from about 600°C to about 800°C. An annealing treatment may soften the strain-hardened composite billet via recrystallization and grain growth. Multiple cold drawing passes and one or more annealing treatments at a suitable temperature (or suitable temperatures) may be employed to form a radiopaque composite wire including a core comprising the rare earth metal, an outer layer comprising the nickel-titanium alloy, and a diffusion barrier comprising the barrier material between the core and the outer layer. Typically, from two to ten cold drawing passes and several interpass annealing treatments are carried out to form the radiopaque composite wire. The number of cold drawing passes may depend on the starting billet size and the final desired wire diameter. A polycrystalline diamond die 208 may be employed for cold drawing with a molybdenum disulphide or other suitable lubricant to reduce the drawing stress.

Generally, at least about 7.5% cold work is imparted to the composite billet during each drawing pass. As would be recognized by one of ordinary skill in the art, percent (%) cold work provides a measurement of the amount of plastic deformation imparted during mechanical working, where the amount is calculated as a percent reduction in a given dimension. In wire drawing, the percent cold work may correspond to the percent reduction in the cross-sectional area of the billet or wire resulting from a drawing pass. The composite billet may undergo multiple cold drawing passes through dies of smaller diameters in order to obtain a desired final wire diameter. The amount of cold work or area reduction imparted to the composite billet to form the composite wire may reach or exceed about 50%. Some or all of the cold drawing passes may be followed by an anneal, as discussed above. Typically, the area reduction with each drawing pass is from about 7.5% to about 15%. For example, a 3 mm diameter becomes 2.77 mm in diameter if a 15% area reduction is achieved. The final cold drawing steps may be the most important, and may be carried out with or without interpass anneals. For example, the final cold drawing passes may involve up to a 50% area reduction to obtain the required dimensions and properties, where multiple smaller area reductions are obtained from successive cold drawing passes without interpass annealing.

The method may further entail fabricating the composite billet which is drawn down to form the composite wire. An ingot comprising the nickel-titanium alloy may be fabricated by melting and casting or powder metallurgy (e.g., spark plasma sintering) methods known in the art, such as those described in U.S. Patent Nos. 9,074,274, 9,103,006 and 9,212,409, which are hereby incorporated by reference. The rod comprising the rare earth metal may also be formed by melting and casting or powder metallurgy methods. A longitudinal hole (bore) may be drilled through the ingot to form the tube comprising the nickel-titanium alloy, and then the barrier layer and the rod comprising the rare earth metal may be assembled in the tube, forming an assembly.

Assembly of the barrier layer and the rod in the tube may comprise wrapping the barrier layer about the rod and inserting the rod and barrier layer into the tube; in this example, the barrier layer may comprise a malleable foil comprising the barrier material. In another embodiment, the assembly may entail, prior to inserting the rod into the tube, coating the barrier layer on the rod (or on an inner wall of the tube) using a vapor or electrochemical deposition process. In another example, the barrier layer may take the form of a hollow cylinder sized to fit over the rod, and assembling the barrier layer and the rod may comprise sliding the rod and the hollow cylinder comprising the barrier material into the tube.

Prior to cold drawing, the assembly may be hot worked (e.g., hot extruded or hot rolled). In one example, the assembly may undergo direct extrusion without a protective sleeve or indirect extrusion canned in a protective copper alloy sleeve at an elevated temperature. Suitable temperatures for hot working are below the melting temperatures of the nickel-titanium alloy (about 1300°C) and the rare earth metal, which may be from about 800°C to about 1500°C, depending on the element. Thus, a hot working (e.g., hot extrusion) temperature in the range from about 500°C to about 1000°C may be suitable. For example, hot extrusion may be carried out at a temperature in the range from 600°C to 800°C. Typical extrusion ratios (calculated by dividing the starting cross-sectional area by the extruded cross-sectional area) may range from at least about 2:1 up to about 27:1, or more typically from 2:1 to about 12:1.

Due to the high temperature exposure during processing, it is advantageous for the composite billet to comprise materials having similar coefficients of thermal expansion. For example, Nitinol is known to have a coefficient of thermal expansion (CTE) of 11 x $10^{-6}$/°C, while Er has a CTE of 12.2 x $10^{-6}$/°C and Lu has a CTE of 9.9 x $10^{-6}$/°C. Otherwise, the core may expand rapidly relative to the outer layer (or vice versa) during processing, possibly inducing rupture of the outer layer or fracture of the core.

The thickness of the barrier layer may be selected depending on the desired thickness of the diffusion barrier in the final composite wire, as well as the anticipated diameter reduction from the starting assembly or composite billet. With each drawing pass, the composite billet is reduced in diameter, and the thickness of the barrier layer decreases also. It is preferred that the diffusion barrier maintains a continuous interface between the outer layer and the core during hot and/or cold working in order to prevent nickel diffusion.

For example, a maximum ratio of the thickness of the barrier layer (e.g., Nb) to the outer diameter of the Ni-Ti alloy tube may be about 1:10 if significant hot working and cold working are required to reduce the composite billet down in size to fine wire. Typically this ratio is closer to 1:33, but it may be a minimum of 1:50. Large composite billets may be reduced by any of the standard hot working methods, e.g. gyratory forging, extrusion, etc. The diffusion barrier can prevent diffusion during hot working as well as during interpass annealing between cold working steps. For production scale processing, the outer diameter of the composite billet is typically about 100 mm in diameter, requiring a barrier layer that is about 3 mm in thickness (1:33).

After cold drawing to form the composite wire, a shape-setting heat treatment may be carried out to impart a "memory" of the desired final shape to the nickel-titanium alloy. The heat treatment may also serve to optimize the properties of the nickel-titanium alloy and alter the phase transformation temperatures. Typically, heat setting temperatures from about 350°C to about 550°C are appropriate to set the final shape and optimize the shape memory/superelastic and mechanical properties of the nickel-titanium alloy. Preferably, the heat setting involves annealing the composite wire while constrained in a final shape at a temperature in the range of from about 350°C to about 550°C. In some cases, heat setting temperatures in the range of from 450°C to 550°C may be appropriate. Heat setting may take place after the composite wire has been incorporated into a medical device using methods known in the art. For example, in order to form a stent, a plurality of radiopaque composite wires may be arranged on a cylindrical mandrel in a braided, woven or other pattern defined by pins protruding radially from the mandrel. After each radiopaque composite wire is wound around the pins as required to form an expanded configuration of the stent, the composite wires may be heat set as described above.

## Radiopacity

The radiopacity of a material is related to its linear absorption coefficient, $\mu$, which depends on its effective atomic number ($Z_{eff}$) and density (p), and on the energy $(E)$ of the incoming x-ray photons:

$$\frac{\mu}{\rho} = const \cdot \frac{Z_{eff}^3}{E^3}$$

The linear absorption coefficient $\mu$ is proportional to the density $\rho$ of the material, and thus the quantity

$$\frac{\mu}{\rho}$$

is a material constant known as the mass absorption coefficient and expressed in units of $cm^2 \, gm^{-1}$. .

Linear absorption coefficients $\mu$ were calculated for several rare earth elements and also for platinum for comparison, and the results are shown in U.S. Patent 9,103,006, which is hereby incorporated by reference. The results indicate that the absorption of the rare earth elements tends to peak in the photon energy range of about 40 to 80 keV, with some rare earth elements exceeding the absorption of platinum in this region.

Magnetic Susceptibility

MRI compatible materials generally have a low magnetic susceptibility. The magnetic susceptibility $X$ of a material can be represented by the ratio of the magnetization M of the material to the applied magnetic field H and is dimensionless:

$$X = \frac{M}{H} \qquad \text{Eq. 1}$$

Different materials respond differently to applied magnetic fields H, and thus may have widely varying values of magnetic susceptibility $X$. As Equation 1 indicates, materials that respond strongly to a magnetic field have a high magnetic susceptibility. Materials are classified as diamagnetic, paramagnetic, or ferromagnetic depending on their response to an applied magnetic field. For example, a susceptibility spectrum may extend from $X = -1.0$ for diamagnetic superconductors to $X > 100,000$ for soft ferromagnetic materials. In the case of medical devices made of materials having a high magnetic susceptibility (such as stainless steel, which is ferromagnetic), a disturbance in the magnetic field is created around the device when the device is visualized with MRI. As a result, the device may not properly transfer MRI signal information and portions of the magnetic resonance image or scan in the vicinity of the component may become distorted, exhibiting dark or black areas in the image. Accordingly, materials having a high magnetic susceptibility are generally not believed to be MRI-compatible.

A material having a magnetic susceptibility X falling in the range from about $-2 \times 10^{-5}$ to about $2 \times 10^{-5}$ may be understood to be MRI compatible. A molar susceptibility $X_m$ is defined as equal to $X \cdot MW/\rho$ ($cm^3/mol$), where MW is molecular weight and $\rho$ is den-

sity. Values of $X_m$ for the rare earth elements are provided in Table 4 below. As can be seen, terbium, dysprosium, holmium, erbium and thulium have relatively high values of molar susceptibility, while rare earth elements such as scandium, yttrium, lanthanum, lutetium, and ytterbium show more promise for applications in which MRI compatibility is important.

*Table 4. Values of Molar Susceptibility for the Rare Earth Elements*

| Rare Earth Element | $X_m/10^{-6} \, cm^3/mol$ |
|---|---|
| Sc | +295.2 |
| Y | +187.7 |
| La | +95.9 |
| Ce | +2,500 |
| Pr | +5,530 |
| Nd | +5,930 |
| Sm | +1,278 |
| Eu | +30,900 |
| Gd | +185,000 |
| Tb | +170,000 |
| Dy | +98,000 |
| Ho | +72,900 |
| Er | +48,000 |
| Tm | +24,700 |
| Yb | +67 |
| Lu | +182.9 |
| Th | +97 |
| Pa | +277 |
| U | +409 |

EXAMPLES

*Radiopaque Composite Wires Including Er and Lu Cores*

A radiopaque composite wire comprising a nickel-titanium outer layer, a rare earth (erbium or lutetium) core, and a niobium diffusion barrier in between is formed by hot rolling followed by cold drawing, as described below.

First, a nickel-titanium alloy tube (56 wt.% Ni-44 wt.%Ti) is fabricated by machining an ingot to an outside diameter of 11.5 mm and boring to a depth of 70 mm to form an inside diameter of 6.5 mm. A rare earth ingot (erbium or lutetium) is machined to a 6 mm outside diameter and a 50 mm length to form a rare earth rod. The rod is wrapped twice in niobium foil of 0.125 mm in thickness and inserted into the bore of the nickel-titanium alloy tube to form an assembly for hot working. The assembly is sealed with a 6.5 mm NiTi plug that extends into the bore to meet the rare earth rod and is TIG welded in place.

The sealed assembly is inserted into a stainless steel protective can (SS321) through a 11.5 mm bore and sealed with a SS321 plug by welding. The can is machined to a square outer cross section before hot rolling in a rolling rig suitable for rolling square profiles. It is rolled at a temperature of 760°C through 7.5% area reductions 40 times. When the protective can is removed, the hot-rolled composite billet measures about 2.5 mm in diameter.

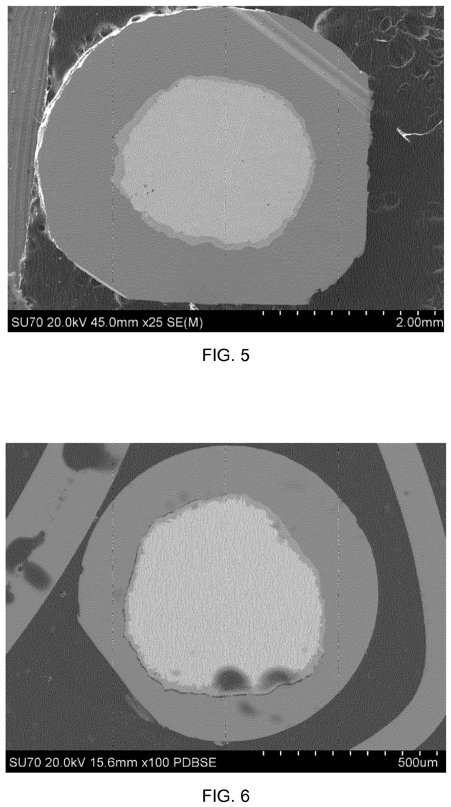

FIG. 3 shows a scanning electron microscope (SEM) image of a hot-rolled composite billet prepared as described above with an erbium rod (core), and FIG. 5 shows a SEM image of a hot rolled composite billet prepared as described above with a lutetium rod (core). Both SEM images show transverse cross-sections of the composite billets. In these particular examples, the starting rare earth ingots were rough cast and underwent manual grinding to the desired size, which explains the roughness apparent in the images. The roughness of the rare earth core can be reduced or eliminated in a manufacturing environment using casting and machining operations known in the art.

Prior to cold drawing, the composite billet undergoes centerless grinding. The composite billet is machined to about 2 mm in diameter, a size selected to ensure the core (Er or Lu rod) is approximately 30 vol.% of the composite. 7.5% area reductions per pass are imparted to the composite billet during each cold drawing pass. The composite billet goes through two passes before annealing at 600°C for 2 minutes, and is pulled through a total of 40 passes to produce a 0.8 mm diameter radiopaque composite wire. For the final drawing passes, the composite wire is pulled through four die reductions without interpass annealing. After cold drawing is completed, the composite wire receives a final straight anneal under tension at 500°C for two minutes to impart superelasticity to the nickel-titanium alloy. This final straight anneal may be referred to as a heat setting treatment.

FIG. 4 shows a transverse cross-sectional SEM image of the cold-rolled composite wire including a nickel-titanium alloy outer layer over an erbium core with a niobium diffusion barrier in between, and FIG. 6 shows a transverse cross-sectional SEM image of the cold-rolled composite wire including a nickel-titanium alloy outer layer over a lutetium core with a niobium diffusion barrier in between.

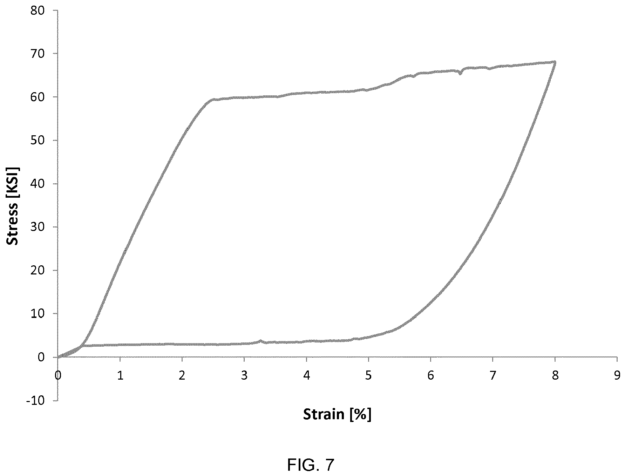

The radiopaque composite wire of the first example (Er core) is subjected to uniaxial tensile testing to evaluate the superelasticity of the composite wire. The wire is tensile tested at room temperature using a 2% per minute strain rate for a 12.7 cm gauge length, which yields an equivalent rate of 2.5 mm/min. Referring to FIG. 7, the tensile testing reveals superelastic behavior with a recoverable strain of about 8%.

Both radiopaque composite wires (Er core and Lu core) undergo x-ray analysis to evaluate their radiopacity in comparison with binary NiTi wires and a commercially available composite wire having a nickel-titanium alloy shell and a platinum core (30 vol.%), described above as DFT wire.

The radiopacity measurements are made on a Ziehm Vision R x-ray imaging system with a voltage and current range of 40-120 kV and 2 mA-72 mA, respectively, for direct radiography. The x-ray tube in the Ziehm Vision R is a dual focus rotating anode tube. A Nuclear Associates Model 07-649 CRDH gastrointestinal (GI) phantom is used. The phantom is produced by Fluke Biomedical based on the Nationwide Evaluation of X-ray Trends (NEXT) survey by the FDA.

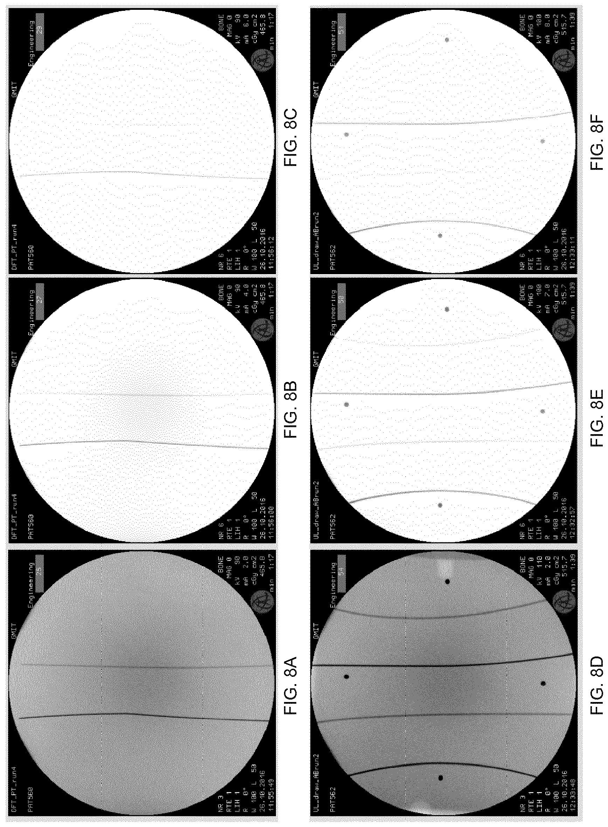

The radiopacity images are in two series shown in FIGs. 8A-8C and 8D-8F, respectively. FIGs. 8A-8C show x-ray images of 0.6 mm-diameter wires of binary NiTi (right) and NiTi - 30 vol.% Pt (left), and FIGs. 8D-8F show the 0.8 mm-diameter radiopaque composite wires fabricated as described above with an Er core (left-most wire) and a Lu core (third wire from left); other wires comprise binary NiTi. A tube voltage of 90 kV is used for the 0.6 mm wires along with a current selected to produce the clearest image. A tube voltage of 100 kV is used for the 0.8 mm wires along with a current again selected to produce the clearest image.

As can be observed, the radiopaque composite wires with the Er and Lu cores exhibit significantly higher x-ray contrast than the binary NiTi wires and have an x-ray contrast higher than or comparable to that of the DFT wire (30 vol.% Pt).

Although the present invention has been described in considerable detail with reference to certain embodiments thereof, other embodiments are possible. The scope of the appended claims should not be limited, therefore, to the description of the preferred embodiments contained herein. All embodiments that come within the meaning of the claims, either literally or by equivalence, are intended to be embraced therein. Furthermore, the advantages described above are not necessarily the only advantages of embodiments of the invention, and it is not necessarily expected that all of the described advantages will be achieved with every embodiment of the invention.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 62/397,123 from which this application claims priority, and in the abstract accompanying this application, are incorporated herein by reference.

**Claims**

1.  A radiopaque composite wire for medical applications, the radiopaque composite wire comprising:

    a core comprising a rare earth metal;
    an outer layer comprising a nickel-titanium alloy disposed over the core; and
    a diffusion barrier comprising a barrier material between the core and the outer layer.

2.  The radiopaque composite wire of claim 1, wherein the barrier material is selected from the group consisting of: a refractory metal, a rare earth oxide, an iron-based material, and carbon.

3.  The radiopaque composite wire of claim 1 or 2, wherein the barrier material comprises a refractory metal selected from the group consisting of: Nb, Mo, Ta, W, Re, Ti, V, Cr, Zr, Hf, Ru, Rh, Os and/or Ir.

4.  The radiopaque composite wire of any preceding claim, wherein the rare earth metal is selected from the group consisting of: Sc, Y, Ce, La, Pr, Nd, Pm, Sm, Eu, Gd, Tb, Dy, Ho, Er, Tm, Yb, Lu, Ac, Th, Pa and U;
    wherein the rare earth metal is preferably selected from the group consisting of: Sc, Y, La, Lu and Yb, the composite wire preferably exhibiting MRI compatibility.

5.  The radiopaque composite wire of any preceding claim,
    wherein the core further comprises an additional element selected from the group consisting of: Ag, Cu, Au, Ir and Rh.

6.  The radiopaque composite wire any preceding claim, wherein the diffusion barrier comprises a thickness of from about 1 micron to about 50 microns, and/or wherein a volume percentage of the core comprising the rare earth metal lies in the range from about 5 vol.% to about 60 vol.%.

7.  A radiopaque medical device comprising:

    at least one radiopaque composite wire according to any preceding claim;
    the radiopaque medical device being an insertable and/or implantable medical device for use in a body vessel.

8.  The radiopaque medical device of claim 7 being selected from the group consisting of: a wire guide, a stent, a stent graft, a torqueable catheter, an introducer sheath, a radiopaque marker or marker band, a grasper, a snare, a basket, a vascular plug, and an embolic protection filter; the radiopaque medical device optionally being a stent selected from the group consisting of: biliary stent, enteral stent, duodenal stent, colonic stent, and esophageal stent.

9.  A method of making a radiopaque composite wire for medical applications, the method comprising:

    cold drawing a composite billet through a die, the composite billet comprising: a tube comprising a nickel-titanium alloy disposed about a rod comprising a rare earth metal, and a barrier layer comprising a barrier material disposed between the tube and the rod;
    after cold drawing, annealing the composite billet to relieve strain, and
    after multiple passes of the cold drawing and annealing, forming a radiopaque composite wire having a core comprising the rare earth metal, an outer layer comprising the nickel-titanium alloy, and a diffusion barrier comprising the barrier material between the core and the outer layer.

10. The method of claim 9, wherein, during cold drawing, at least about 7.5% cold work is imparted to the composite billet; and/or wherein the method comprises, prior to cold drawing the composite billet, hot working the composite billet.

11. The method of claim 10, comprising, prior to cold drawing the composite billet, hot working the composite billet, and, prior to hot working the composite billet, fabricating the composite billet, wherein fabricating the composite billet comprises:

    drilling a longitudinal hole through an ingot comprising the nickel-titanium alloy to form the tube; and
    assembling the barrier layer comprising the barrier material and the rod comprising the rare earth metal in the tube.

12. The method of claim 11, wherein assembling the barrier layer and the rod in the tube comprises:

    wrapping the barrier layer about the rod, the barrier layer comprising a foil, and
    inserting the barrier layer and rod into the tube.

13. The method of claim 11 or 12, wherein assembling the barrier layer and the rod in the tube comprises:

    applying the barrier layer onto the rod using a vapor or electrochemical deposition process, and
    inserting the rod coated with the barrier layer into the tube.

14. The method of any of the claims 11 to 13, wherein assembling the barrier layer and the rod in the tube comprises:

inserting the rod into the tube; and
inserting a hollow cylinder comprising the barrier material into the tube.

15. The method of any of claims 11 to 14, wherein assembling the barrier layer and the rod in the tube comprises:

applying the barrier layer onto an inner wall of the tube using a vapor or electrochemical deposition process, and inserting the rod into the tube.

FIG. 1

FIG. 2

FIG. 3

FIG. 4

FIG. 5

FIG. 6

FIG. 7

FIG. 8A

FIG. 8B

FIG. 8C

FIG. 8D

FIG. 8E

FIG. 8F

FIG. 9

Europäisches
Patentamt

European
Patent Office

Office européen
des brevets

**EUROPEAN SEARCH REPORT**

Application Number

EP 17 27 5145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/154666 A1 (MEDTRONIC VASCULAR INC [US]) 17 October 2013 (2013-10-17)<br>* paragraph [[0009]] - paragraph [[0010]] *<br>* paragraph [[0049]] - paragraph [[0051]] *<br>* paragraph [[0065]] - paragraph [[0067]] *<br>----- | 1-15 | INV.<br>A61L31/02<br>A61L31/08<br>A61L29/02<br>A61L29/10<br>A61L29/12<br>A61L31/12<br>A61L29/18<br>A61L31/18 |
| A | US 8 871 829 B2 (BODO GEROLD ET AL.) 28 October 2014 (2014-10-28)<br>* column 4, line 49 - line 57 *<br>* column 5, line 23 - line 41; claims *<br>----- | 1-8 | |
| A | EP 1 070 558 A2 (BRIDGESTONE CORP [JP]) 24 January 2001 (2001-01-24)<br>* claims *<br>----- | 9-15 | |
| A | US 2009/177268 A1 (LUNDKVIST ANDRE S [US] ET AL) 9 July 2009 (2009-07-09)<br>* paragraphs [[0010]], [[0013]]; claims *<br>----- | 1-15 | TECHNICAL FIELDS SEARCHED (IPC) |
| A | EP 1 938 776 A1 (BOSTON SCIENT LTD [BB]) 2 July 2008 (2008-07-02)<br>* paragraph [[0009]] - paragraph [[0014]] *<br>* paragraphs [[0039]], [[0042]], [[0069]], [[0072]]; claims *<br>----- | 1-15 | A61L |
| A | EP 2 768 993 B1 (UNIV LIMERICK [IE]) 13 April 2016 (2016-04-13)<br>* paragraphs [[0014]], [[0020]] - [[0026]], [[0039]], [[0040]] *<br>* paragraph [[0050]] - paragraph [[0052]] *<br>* paragraph [[0054]] *<br>----- | 1-15 | |

-/--

The present search report has been drawn up for all claims

| Place of search | Date of completion of the search | Examiner |
|---|---|---|
| The Hague | 5 February 2018 | Espinosa y Carretero |

EPO FORM 1503 03.82 (P04C01)

page 1 of 2

Europäisches
Patentamt

European
Patent Office

Office européen
des brevets

# EUROPEAN SEARCH REPORT

Application Number

EP 17 27 5145

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 059 619 B1 (COOK INC [US]; COOK IRELAND LTD [IE]) 17 November 2010 (2010-11-17) * paragraph [[0047]] - paragraph [[0048]] * * paragraph [[0128]] - paragraph [[0133]]; examples * ----- | 1-15 | |

TECHNICAL FIELDS
SEARCHED       (IPC)

The present search report has been drawn up for all claims

| Place of search | Date of completion of the search | Examiner |
|---|---|---|
| The Hague | 5 February 2018 | Espinosa y Carretero |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 27 5145

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2018

| Patent document cited in search report | Publication date | Patent family member(s) | Publication date |
|---|---|---|---|
| WO 2013154666 A1 | 17-10-2013 | US 2013274864 A1<br>US 2017216060 A1<br>WO 2013154666 A1 | 17-10-2013<br>03-08-2017<br>17-10-2013 |
| US 8871829 B2 | 28-10-2014 | DE 10361942 A1<br>EP 1696978 A1<br>US 2007191708 A1<br>WO 2005065737 A1 | 21-07-2005<br>06-09-2006<br>16-08-2007<br>21-07-2005 |
| EP 1070558 A2 | 24-01-2001 | EP 1070558 A2<br>JP 2001025813 A | 24-01-2001<br>30-01-2001 |
| US 2009177268 A1 | 09-07-2009 | CA 2711484 A1<br>CA 2951303 A1<br>CN 101969891 A<br>EP 2247268 A2<br>ES 2585404 T3<br>JP 5419893 B2<br>JP 2011509124 A<br>US 2009177268 A1<br>US 2010152837 A1<br>US 2014058500 A1<br>US 2014082924 A1<br>WO 2009089216 A2<br>WO 2009089218 A2 | 16-07-2009<br>16-07-2009<br>09-02-2011<br>10-11-2010<br>05-10-2016<br>19-02-2014<br>24-03-2011<br>09-07-2009<br>17-06-2010<br>27-02-2014<br>27-03-2014<br>16-07-2009<br>16-07-2009 |
| EP 1938776 A1 | 02-07-2008 | AT 392197 T<br>AU 2003272774 A1<br>CA 2500711 A1<br>DE 60320430 T2<br>EP 1549250 A2<br>EP 1938776 A1<br>JP 2006501032 A<br>US 6638301 B1<br>US 2004068315 A1<br>US 2007190231 A1<br>US 2010174360 A1<br>US 2011257730 A1<br>WO 2004030578 A2 | 15-05-2008<br>23-04-2004<br>15-04-2004<br>28-05-2009<br>06-07-2005<br>02-07-2008<br>12-01-2006<br>28-10-2003<br>08-04-2004<br>16-08-2007<br>08-07-2010<br>20-10-2011<br>15-04-2004 |
| EP 2768993 B1 | 13-04-2016 | CN 103906850 A<br>EP 2768993 A1<br>GB 2495772 A<br>JP 6177784 B2<br>JP 2014534342 A<br>JP 2017201059 A | 02-07-2014<br>27-08-2014<br>24-04-2013<br>09-08-2017<br>18-12-2014<br>09-11-2017 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 17 27 5145

This annex lists the patent family members relating to the patent documents cited in the above-mentioned European search report.
The members are as contained in the European Patent Office EDP file on
The European Patent Office is in no way liable for these particulars which are merely given for the purpose of information.

05-02-2018

| Patent document cited in search report | Publication date | Patent family member(s) | Publication date |
|---|---|---|---|
| | | US 2013101455 A1 | 25-04-2013 |
| | | WO 2013057292 A1 | 25-04-2013 |
| EP 2059619 B1 | 17-11-2010 | AT 488611 T | 15-12-2010 |
| | | AU 2007293025 A1 | 13-03-2008 |
| | | CA 2658580 A1 | 13-03-2008 |
| | | DK 2059619 T3 | 21-02-2011 |
| | | EP 2059619 A1 | 20-05-2009 |
| | | JP 5748955 B2 | 15-07-2015 |
| | | JP 2010502842 A | 28-01-2010 |
| | | US 2008053577 A1 | 06-03-2008 |
| | | US 2015232975 A1 | 20-08-2015 |
| | | WO 2008030517 A1 | 13-03-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9074274 B
- US 9103006 B
- US 9212409 B
- US 62397123 B